# EUROPEAN PATENT SPECIFICATION

(11) **EP 2 395 949 B1**
(45) Date of publication and mention of the grant of the patent: **05.06.2013**
(21) Application number: 10722803.3
(22) Date of filing: 12.02.2010
(51) Int. Cl.: A61F 2/46, A61B 17/88, A61B 18/04

(54) **HEATED TIP IMPLANT DELIVERY SYSTEM**
IMPLANTATFREISETZUNGSSYSTEM MIT ERWÄRMTEN SPITZEN
SYSTÈME D'ADMINISTRATION D'UN IMPLANT À EXTRÉMITÉ CHAUFFANTE

(30) Priority: 13.02.2009 US 152392 P
(43) Date of publication of application: 21.12.2011
(73) Proprietor: Warsaw Orthopedic, Inc., Warsaw, Indiana 46581 (US)
(72) Inventor: WINTERBOTTOM, John, M., Howell NJ 07731 (US); KAES, David, R., Toms River NJ 08753 (US)
(74) Representative: Hally, Anna-Louise
(86) International application number: PCT/US2010/024117
(87) International publication number: WO 2010/093935

(56) References cited:
- US-A- 4 357 136
- US-A1- 2009 012 525
- US-B1- 6 436 143

## Description

### Field of the Invention

This invention pertains to a delivery system for heating and delivering a bone substitute material, such as a bone/polymer composite that is made flowable upon heating. The closest prior art is US 2009/0012525 A1, which discloses the preamble of claim 1.

### Background of the Invention

Bone is a composite material composed of impure hydroxyapatite, collagen, and a variety of non-collagenous proteins, as well as embedded and adherent cells. Bone can be processed into an implantable biomaterial, such as an allograft. The processed bone biomaterial can have a variety of properties depending upon the specific processes and treatments applied to it, and it may incorporate characteristics of other biomaterials (e.g., polymers) with which it is combined. For example, bone-derived biomaterials may be processed into bone/polymer composites that have the ability to induce a cellular healing response.

The use of bone grafts and bone substitute materials in orthopedic medicine is well known. While bone wounds can regenerate without the formation of scar tissue, fractures and other orthopedic injuries require a long time to heal, during which the injured bone is unable to support physiologic loading. Metal pins, screws, and meshes are frequently needed to replace or supplement the mechanical function of the injured bone. However, metal is significantly stiffer than bone. Use of metal implants sometimes results in decreased bone density around the implant site due to stress shielding. Furthermore, most metal implants are permanent and unable to participate in physiological remodeling.

Bone's cellular healing processes, through bone tissue formation by osteoblast cells coordinated with bone and graft resorption by osteoclast cells, permit bone grafts and certain bone substitute materials to remodel into endogenous bone that is almost indistinguishable from the original. However, the use of bone grafts is limited by the available shape and size of grafts and the desire to optimize both mechanical strength and degradation rate. Variations in bone size and shape among patients (and donors) also make bone grafts a less optimal substitute material.

Thus, bone substitute materials such as bone particle/polymer composites have been developed that can be made moldable or flowable at elevated temperatures. Such composites require an efficient system for conveniently heating the material to a moldable temperature in a sterile operating room and then delivering the material.

### Summary of the Invention

The present invention is defined in claim 1. The present invention stems from the recognition that certain bone substitute material (e.g., bone/polymer composites, thermoplastic polymers, thermoplastic composites) must be heated to make it moldable and/or flowable. The heated, moldable or flowable material can then be used to fill irregularly shaped volumes in or near a bone such as a fracture, void, bone fusion, or iatrogenic bony defect. Such material can also be used to immobilize fragments of a comminuted fracture. In certain embodiments, the temperature of the material may be such that a necrotic ring forms around the implantation site, such as when tumor cells or carcinogenic cells are to be destroyed before bone healing can begin. A bone substitute material that is moldable while being implanted but later becoming set with a desired degree of mechanical strength would be particularly useful in treating bony defects or creating bone fusions in a subject. The bone substitute material could be molded, shaped, extruded, or injected into the site of implantation and then be allowed to set as it cools to body temperature. In some embodiments, the set material provides the desired mechanical strength for the implantation site reducing the need for metal pins, screws, or meshes. Unlike metal, the bone substitute material may be bioresorbable to allow for growth of new bone into the site. The material may actually promote the growth of new bone, for example, by releasing a growth factor or anabolic agent. The present invention provides a system for heating and administering a bone substitute material, such as a bone/polymer composite, in a flowable state. The inventive system includes a device with a heated tip for delivering a heated flowable bone substitute material, which heats the bone substitute material as it exits the tip.

In one aspect, the invention provides a device that comprises a cannula with an attached or embedded heating apparatus that delivers a sufficient amount of heat to make the material (*e.g*., bone substitute material) passing through it flowable, wherein at least the distal tip of the cannula is heated. In certain embodiments, heating of the material progresses in two or more stages to make the material initially flowable at a first elevated temperature and then heated to a second elevated temperature to allow delivery to the implantation site. These regions of the device may have separate controls or may be controlled by a single setting to be within a defined temperature relationship. Material is typically fed into one end of the cannula in an unheated state. As the material advances through a heated portion of the cannula, it is heated to a temperature at which the material becomes moldable or flowable (*e.g.*, from about 45 °C to about 80 °C). The material is advanced through the cannula by either the direct application of a mechanical force (*e.g*., a syringe-type plunger or auger) or the application of pressure from a pressurized gas or other fluid. The amount of heat delivered to the material may be regulated directly by the user (*e.g*., using a thermostat) and/or indirectly by the amount of time the material spends in contact with the heated cannula (*e.g*., controlling the amount of pressure applied to the material). A feedback control system may be used to maintain the temperature within a desired range, within a useable range, or with a range that renders the material flowable. The heated tip of the cannula may also be used to remelt or remodel material that has already been administered. The heat applied may provide for antibacterial, antimicrobial, and/or sterilization effects. The cannula and other components of the device in contact with the material may be coated so that the material does not stick to them. For example, the coating may be TEFLON^{®} or another polymeric or hydrophobic material. The tip of the cannula may be interchangeable to accommodate different sizes and shapes of tips (*e.g.*, wide, narrow, high gauge, low gauge, oval, round, rectangular, square, flattened, *etc*.). In certain embodiments, the heated cannula is a component of a delivery device with a handle, casing, barrel, chamber for the material to be delivered, and trigger to administer the material. The tip may be configured to provide a pre-measured volume of material to the implantation site and then to halt the flow of material.

The device may be similar in design to a glue gun or cookie press. The material to be heated and administered may be provided in pellets, beads, sticks, or blocks of various dimensions and characteristics. The stick or blocks of material may be similar to glue sticks that are sold for glue guns. The material may be provided in a cartridge, which can be easily loaded into the device. The device typically comprises a passage, chamber, or compartment for loading the material into the gun, a plunger or trigger for applying pressure to the material to advance the material through the device, and a heated tip or cannula for heating the material to a flowable state. The device may also include certain controls, for example, an on/off switch, a thermostat for regulating the temperature of the tip, and/or a control for adjusting how quickly the material is delivered by the device. The device may also be designed with a comfortable grip for holding and using the device. Power may be provided to the device using a power cord or a battery. The device may have a fiber optic cable or charge coupled device for delivering light to the implantation site or for visualizing the site. Furthermore, the device may include non-functional design elements such as names, logos, lettering, casing, and coloring. The device may be reusable or disposable after one use. The device may comprise a cartridge that is disposable after one use and an applicator that is reuseable. The device may also be designed to accommodate use with other materials, such as antibiotics or other pharmaceutical agents, autografts, reinforcing fibers, bone substitute materials, etc., which may optionally be combined with the heated, flowable material. For a multiple use device, the device is able to be sterilized.

The invention may be used to heat a bone substitute material to a temperature ranging from about 45 °C to about 80 °C using an inventive device so that the material becomes flowable and can be delivered through a heated cannula or tip as described herein or a delivery device (e.g., a glue gun-like or cookie press device) as described herein. Exemplary bone substitute materials that can be heated using the inventive devices include composites having a bone, mineral, or ceramic component and a polymer component. Examples of such bone/polymer composites are described in published U.S. patent applications, US 2005/0008672, published January 13, 2005; US 2007/0191963, published August 16, 2007; and US 2008/0069852, published March 20, 2008. The material may be a thermoplastic composite. The material may also be a thermoplastic polymer. The bone substitute material is loaded into an inventive device and heated to a specified temperature as it is advanced through the heated cannula, tip, or other device. The heated material is delivered to an implantation site (*e.g*., bony defect, joint to be fused, bone segments or fragments to be united, *etc.)* of a subject using the device. Therefore, heat is applied to the material just as it is being delivered. The steps may be performed in a sterile environment such as an operating room. The bone substitute materials may be provided packaged sterilely and conveniently for loading into a hot melt delivery device as described herein. The material may be packaged, and the packaging is removed only prior to loading of an inventive device. Given that the bone substitute material is frequently used in a surgical setting, the method preferably provides sterile, flowable material in a short span of time.

In another aspect, the invention provides a kit that may include the bone substitute materials as well as the inventive device for heating and administering the material. The material and device may be packaged conveniently for use in a sterile environment. The kit may include instructions for using the material and/or the device. The kit may include multiple samples of the bone substitute material. Each sample may be individually packaged. The kit may include tools or other materials for manipulating or using the bone substitute material. The kit may include pharmaceutical agents that may be used with the bone substitute material and/or during the implantation of the bone substitute material. Exemplary pharmaceutical agents may include antibiotics, osteoinductive factors, osteoconductive factors, osteogenic factors, growth factors, anesthetics, angiogenic factors, anti-inflammatory agents, adhesives, vasoconstrictors, *etc*. The kit may be for one use or multiple uses.

### Brief Description of the Drawing

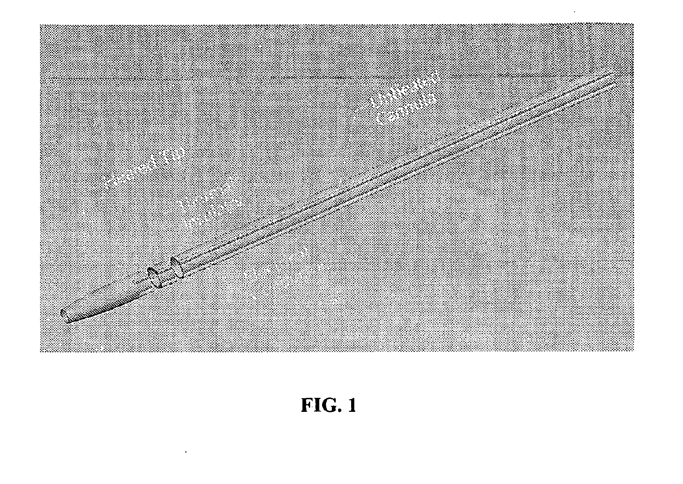

*Figure 1* shows a component view of an exemplary design of an inventive cannula with a heated tip.

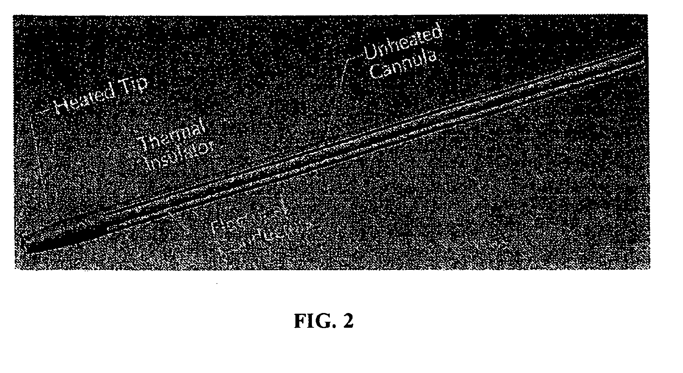

*Figure 2* shows an assembled view of an exemplary design of an inventive cannula with a heated tip.

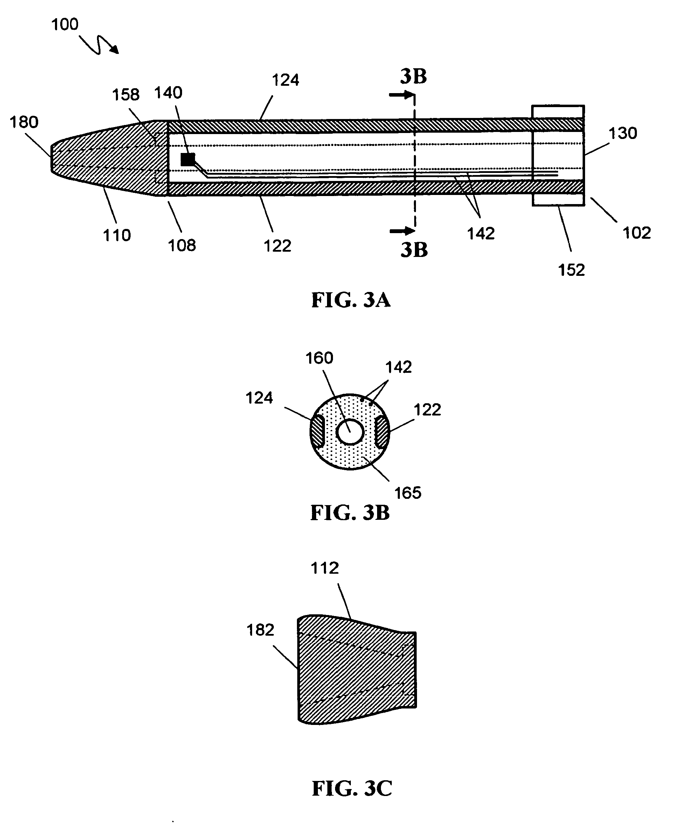

*Figure 3A* is a schematic of an inventive heated tip cannula (100) for delivery of a heated bone substitute material. The cannula has a near or inlet end (102) and a distal or outlet end (108). The cannula (100) also includes a removable and interchangeable tip (110) that comprises resistive heating material, a conductive element (122) for providing electrical current to the tip and a conductive element (124) for receiving electrical current from the tip. Material can be provided at an inlet orifice (130) to hollow core of the cannula while material can be applied to subject from an outlet orifice (180) from hollow core of the cannula. The orifice (180) can be round, square, rectangular, or oval. The cannula may also include a temperature sensing element (140) (e.g., a thermistor, used to monitor temperature in the vicinity of the tip (110). Electrical cables/wires (142) leading to the temperature sensing element (140) may be deposited on the outside of the cannula or embedded within the cannula. Fastening features (152 and 158) (e.g., threads, bayonet-type fastener, twist-and-lock, quick release fastener, etc.) can enable the cannula to be fastened securely to and removed from a gun body or enable the tip to be fastened securely to and removed from the cannula. *Figure 3B* shows a cross-sectional view of the cannula (100) with hollow core (160) of the cannula in the center and the heating elements (122 and 124) on the sides of the body of cannula (165). *Figure 3C* is an alternative embodiment of the tip (112). Such a tip design can be used to deliver a sheet of material. The outlet orifice (182) can be a long think opening for applying a "sheet" of material.

*Figure 4* is a schematic of an exemplary delivery device (200). The device includes a body (210), heated tip cannula (100), a grip (220), user controls (232, 234, 236), power cord (260), and chamber (240) for the bone substitute material to be delivered.

### Definitions

The term *"cannula"* as used herein refers to a hollow tube that can be inserted into a site *(e.g.,* a bony defect) for delivering a bone substitute material. The cannula may be flexible or stiff. In certain embodiments, a portion of the cannula (e.g., the tip) is heated.

The term *"composite"* is used to refer to a unified combination of two or more distinct materials. The composite may be homogeneous or heterogeneous. For example, a composite may be a combination of bone-derived particles and a polymer; or a combination of a ceramic or mineral, and a polymer.

As used herein, the term *"flowable material"* refers to a composition that has been rendered flowable through a cannula or tip of an inventive device as described herein. In some embodiments, flowable materials include polymers or composites that have been heated past their glass transition or melting point. The flowable material may be heated to a temperature sufficient to cause a chemical change in the material (*e.g*., polymerization or cross-linking of the material).

As used herein, *"flowable"* materials are those that can be advanced through a tube or hole. The flowable material may be heated to make it flowable, and pressure may be need to be applied to advance the flowable materials through the hole or tube. The lumen or hole through which the flowable material is advanced may be at least 0.25 cm in diameter.

As used herein, the term *"glass transition temperature"* (T_{g}) indicates the lowest temperature at which an amorphous or partially amorphous polymer is considered softened and possibly flowable. As referred to herein, the value of T_{g} is to be determined using differential calorimetry as per ASTM Standard E1356-98 "Standard Test Method for Assignment of the Glass Transition Temperatures by Differential Scanning Calorimetry or Differential Thermal Analysis."

As used herein, the term *"melting temperature"* (Tₘ) is defined as the temperature, at atmospheric pressure, at which a polymer transitions from a crystalline state to a viscous flow state. As referred to herein, the value of Tₘ is the value of Tₚₘ₁ as determined according to per ASTM Standard D3418-99 "Standard Test Method for Transition Temperatures of Polymers By Differential Scanning Calorimetry."

The term *"osteoconductive",* as used herein, refers to the ability of a substance or material to provide surfaces which are receptive to the growth of new bone.

The term *"osteogenic"* refers to the ability of a substance or material that can induce bone formation.

*"Osteoinductive",* as used herein, refers to the quality of being able to recruit cells (e.g., osteoblasts) from the host that have the potential to stimulate new bone formation. In general, osteoinductive materials are capable of inducing heterotopic ossification, that is, bone formation in extraskeletal soft tissues (*e.g*., muscle).

The term *"osteoimplant"* is used herein in its broadest sense and is not intended to be limited to any particular shapes, sizes, configurations, compositions, or applications. Osteoimplant refers to any device or material for implantation that aids or augments bone formation or healing. Osteoimplants are often applied at a bone defect site, *e.g*., one resulting from injury, defect brought about during the course of surgery, infection, malignancy, inflammation, or developmental malformation. Osteoimplants can be used in a variety of orthopedic, neurosurgical, dental, and oral and maxillofacial surgical procedures such as the repair of simple and compound fractures and non-unions, external, and internal fixations, joint reconstructions such as arthrodesis, general arthroplasty, deficit filling, disectomy, laminectomy, anterior cerival and thoracic operations, spinal fusions, *etc.* The osteoimplant may be made from a moldable bone substitute material or from a moldable bone particle/polymer composite.

The term *"penetrate"* refers to the ability of one substance to invade or infiltrate another. Penetrate may refer to complete or partial penetration. A polymer may infiltrate the particles of the composite. That is, the polymer may infiltrate the voids, gaps, holes, pores, crevices, *etc.* of the particles. After implantation, cells, tissue, or bone may invade the implanted composite. Also, a moldable composite may penetrate the voids, gaps, holes, pores, crevices, *etc.* of an implantation site.

As used herein, *"pharmaceutical agent"* is used to refer to compounds or entities that alter, promote, speed, prolong, inhibit, activate, or otherwise affect biological or chemical events in a subject (e.g., a human). For example, pharmaceutical agents may include, but are not limited to, osteogenic, osteoinductive, and osteoconductive agents, anti-HIV substances, anti-cancer substances, antibiotics, immunosuppressants, anti-viral agents, enzyme inhibitors, neurotoxins, opioids, hypnotics, anti-histamines, lubricants, muscle relaxants, anti-spasmodics and muscle contractants including channel blockers, miotics and anti-cholinergics, anti-parasite agents, anti-protozoal agents, and anti-fungal agents, modulators of cell-extracellular matrix interactions including cell growth inhibitors and anti-adhesion molecules, vasodilating agents, vasoconstricting agents, inhibitors of DNA, RNA, or protein synthesis, anti-hypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, prostaglandins, antidepressants, anti-psychotics, targeting agents, chemotactic factors, receptors, neurotransmitters, proteins, cell response modifiers, cells, peptides, polynucleotides, viruses, and vaccines. In certain examples, the pharmaceutical agent is a drug. In certain examples, the pharmaceutical agent is a small molecule.

A more complete listing of pharmaceutical agents and specific drugs suitable for use in the present invention may be found in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", Edited by Susan Budavari et al., CRC Press, 1996, the United States Pharmacopeia-25/National Formulary-20, published by the United States Pharmcopeial Convention, Inc., Rockville MD, 2001, and the "Pharmazeutische Wirkstoffe", edited by Von Keemann et al., Stuttgart/New York, 1987. Drugs for human use listed by the U.S. Food and Drug Administration (FDA) under 21 C.F.R. §§ 330.5, 331 through 361, and 440 through 460, and drugs for veterinary use listed by the FDA under 21 C.F.R. §§ 500 through 589, are also considered acceptable for use in accordance with the present invention.

The terms *"polynucleotide", "nucleic acid",* or *"oligonucleotide"* refer to a polymer of nucleotides. The terms "polynucleotide", "nucleic acid", and "oligonucleotide", may be used interchangeably. Typically, a polynucleotide comprises at least three nucleotides. DNAs and RNAs are exemplary polynucleotides. The polymer may include natural nucleosides (i.e., adenosine, thymidine, guanosine, cytidine, uridine, deoxyadenosine, deoxythymidine, deoxyguanosine, and deoxycytidine), nucleoside analogs (e.g., 2-aminoadenosine, 2-thithymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g.*, methylated bases), intercalated bases, modified sugars *(e.g.,* 2'-fluororibose, ribose, 2'-deoxyriboses, arabinose, and hexose), or modified phosphate groups (e.g., phosphorothioates and 5'-N-phosphoramidite linkages). The polymer may also be a short strand of nucleic acids such as RNAi, siRNA, or shRNA.

As used herein, a *"polypeptide", "peptide",* or *"protein"* includes a string of at least three amino acids linked together by peptide bonds. The terms, "polypeptide", "peptide", and "protein", may be used interchangeably. Some peptides may contain only natural amino acids, although non-natural amino acids (*i.e.*, compounds that do not occur in nature but that can be incorporated into a polypeptide chain) and/or amino acid analogs as are known in the art may alternatively be employed. Also, one or more of the amino acids in a peptide may be modified, for example, by the addition of a chemical entity such as a carbohydrate group, a phosphate group, a farnesyl group, an isofarnesyl group, a fatty acid group, a linker for conjugation, functionalization, or other modification, *etc.* Modifications of the peptide may lead to a more stable peptide (e.g., greater half-life *in vivo*). These modifications may include cyclization of the peptide, the incorporation of D-amino acids, *etc.* None of the modifications should substantially interfere with the desired biological activity of the peptide.

The term *"porogen"* refers to a chemical compound that may be part of the inventive composite and upon implantation or prior to implantation diffuses, dissolves, and/or degrades to leave a pore in the osteoimplant composite. The porogen may be introduced into the composite during manufacture, during preparation of the composite (e.g., in the operating room), or after implantation. The porogen essentially reserves space in the composite while the composite is being molded but once the composite is implanted the porogen diffuses, dissolves, or degrades, thereby inducing porosity into the composite. In this way the porogen provides latent pores. The porogen may also be leached out of the composite before implantation. This resulting porosity of the implant generated during manufacture or after implantation (*i.e.,* "latent porosity") is thought to allow infiltration by cells, bone formation, bone remodeling, osteoinduction, osteoconduction, and/or faster degradation of the osteoimplant. A porogen may be a gas (e.g., carbon dioxide, nitrogen, or other inert gas), liquid (*e.g*., water, biological fluid), or solid. Porogens are typically water soluble such as salts, sugars (*e.g*., sugar alcohols), polysaccharides *(e.g.,* dextran (poly(dextrose)), water soluble small molecules, *etc.* Porogen can also be natural or synthetic polymers, oligomers, or monomers that are water soluble or degrade quickly under physiological conditions. Exemplary polymers include polyethylene glycol, poly(vinylpyrollidone), pullulan, poly(glycolide), poly(lactide), poly(lactide-co-glycolide), other polyesters, and starches.

The terms *"porosity"* and *"void volume"* are used herein interchangeably and refer to the average amount of non-solid space contained in a material (e.g., a composite of the present invention). Such space is considered void of volume even if it contains a substance that is liquid at ambient or physiological temperature, e.g., 0.5 °C to 50 °C. The porosity or void volume of a composite can be defined as the ratio of the total volume of the pores (i.e., void volume) in the material to the overall volume of the composite. Porosity may in certain embodiments refer to "latent porosity" wherein pores are only formed upon diffusion, dissolution, or degradation of a material occupying the pores. The pores in such an instance may be formed after implantation.

As used herein, the term *"settable"* refers to a material that may be rendered more resistant to mechanical deformation as compared to a formable state. In certain examples, the material becomes set as it cools to body temperature.

As used herein, the term *"set"* refers to the state of a material that has been rendered more resistant to mechanical deformation with respect to a formable state.

The term *"shaped"* as used to characterize a material *(e.g.,* composite) or an osteoimplant refers to a material or osteoimplant of a determined or regular form or configuration in contrast to an indeterminate or vague form or configuration (as in the case of a lump or other solid matrix of special form). The material may be shaped into any shape, configuration, or size. Materials can be shaped as sheets, blocks, plates, disks, cones, pins, screws, tubes, teeth, bones, portions of bones, wedges, cylinders, threaded cylinders, and the like, as well as more complex geometric configurations.

As used herein, the term *"small molecule"* is used to refer to molecules, whether naturally-occurring or artificially created (*e.g*., via chemical synthesis), that have a relatively low molecular weight. Typically, small molecules have a molecular weight of less than about 2,500 g/mol, more preferably less than 1,000 g/mol. Preferred small molecules are biologically active in that they produce a local or systemic effect in animals, preferably mammals, more preferably humans. The small molecule may be a drug. Preferably, though not necessarily, the drug is one that has already been deemed safe and effective for use by an appropriate governmental agency or body (*e.g*., the U.S. Food and Drug Administration).

The term *"thermoplastic",* as used herein, refers to the property of a material being soft at an elevated temperature, rigid upon cooling, and soft again upon re-heating. That is, the process is reversible. Thermoplastic materials, unlike thermosetting materials, can be remelted and remolded.

As used herein, the term *"transformation"* describes the process by which a material is removed from an implant site and replaced by host tissue after implantation. Transformation may be accomplished by a combination of processes, including but not limited to remodeling, degradation, resorption, and tissue growth and/or formation. Removal of the material may be cell-mediated or accomplished through chemical processes, such as dissolution and hydrolysis.

### Detailed Description of Certain Embodiments of the Invention

The present invention stems from the recognition that it would be useful to be able to conveniently heat bone substitute materials that become flowable at elevated temperatures in a device that could also be used to administer the heated material. Such a device would heat the material using the heated tip of a cannula or a device similar to a hot melt glue gun. The device would heat the material as it is advanced through the heated cannula or tip; therefore, the material would be heated just as it is being delivered. This would avoid heating the material for an unnecessarily long period of time before administration and therefore prevent the degradation of biological components (e.g., proteins, cells) of the material. The invention also provides methods of using the inventive device to heat bone substitute materials. Kits that include the device and materials for use in the device are also provided by the present invention.

Bone substitute materials such as bone/polymer composites, mineral/polymer composites, and ceramic/polymer composites have been developed that are moldable or flowable at elevated temperatures. The composites typically include bone particles in a polymer phase (e.g., polycaprolactone, poly(lactide-co-glycolide), polyurethane). The composites become moldable or flowable when heated to a temperature ranging from about 70 °C to about 95 °C. Being moldable or flowable, the composite can be easily administered to an irregular or hard-to-access site and can also conform to the implantation site. Once the composite cools to body temperature, it becomes set and can bear physiological loading. Such composites may be used in a variety of orthopedic and dental applications. Any composite useful in orthopedic or dental applications that softens upon heating may be utilized in the present invention with the inventive devices. The composite may include a porogen (e.g., a gas, salt, carbohydrate, *etc*.). The composite may include a pharmaceutical agent. Exemplary composites that are moldable at elevated temperatures are described in U.S. provisional patent application, USSN 60/432,968, filed December 12, 2002; U.S. patent application, USSN 10/735,135, filed December 12, 2003, published as US 2005/0008672 on January 13, 2005; international PCT application, WO 04/53112, published June 24, 2004; U.S. provisional patent application, USSN 60/760,538, filed January 19, 2006; international PCT application, WO 2007/084725, published July 26, 2007; U.S. patent application, USSN 11/625,119, filed January 19, 2007, published as US 2007/0191963 on August 16, 2007; U.S. patent application, USSN 11/625,086, filed January 19, 2007, published as US 2008/0069852 on March 20, 2008.

### Heating Devices

The present invention provides heating devices especially designed for heating and administering bone substitute materials or thermoplastic materials. Typically the inventive heating devices are designed to conveniently and sterilely heat a sample of material just as it is delivered to the implant site. The device typically includes a cannula with a heated tip; therefore, as the material is advanced through the heated tip of the cannula, it becomes flowable and therefore easy to deliver to an implant site. The heated cannula tip may be used as part of a larger device with a casing, user controls, grip, battery or power supply, compartment for the material to be delivered, *etc.* In certain embodiments, the inventing device is similar in design to a hot melt glue gun. The heating device is typically designed and constructed to allow for sterilization of the device.

The heating device comprises a cannula for delivering a material that is made flowable by heating. The cannula includes a portion that is heated so as to heat the material as it is advanced through the cannula to a temperature sufficient to make the material flowable. According to the invention, the heating apparatus is removable. For example, the heating apparatus may be removed from cannula and positioned on another cannula. In this way, the cannula may be disposable, and the heating apparatus may be reusable. Or the cannula may be cleaned and sterilized separately from the heating apparatus. The heating apparatus is heated via a power source such as an electrical cord or battery. In the US, the heating apparatus may be designed to plug into a standard 110 V outlet. Outside the US, the heating apparatus may be designed to plug into a 220 V outlet. The heating device may have an initial setting in which it sterilizes the inner chamber and tip before the cannula is used to administer flowable material or come in contact with the implantation site. The heating apparatus typically comes with a medical grade cord suitable for plugging the device into an electrical outlet.

Heat is generated in the heating apparatus by resistive heating. The heat generated by the heating apparatus is transferred to the walls of the cannula by conduction and/or convection. The walls of the cannula may be constructed of a material known to conduct heat such as a metal (*e.g*., stainless steel, titanium). The amount of heat generated by the heating apparatus may be controlled by a thermostat. The heating apparatus is typically kept at a temperature ranging from approximately 50 °C to approximately 120 °C. In certain embodiments, the heating apparatus is kept at a temperature ranging from approximately 60 °C to approximately 100 °C. In certain embodiments, the heating apparatus is kept at a temperature ranging from approximately 60 °C to approximately 80 °C. In some embodiments, the heating apparatus is kept at a temperature ranging from about 45 °C to about 130 °C, from about 45 °C to about 120 °C, from 45 °C to about 110 °C, from about 45 °C to about 90 °C, or from about 45 °C to about 80 °C. In some embodiments, the heat apparatus is kept at a temperature ranging from about 75 °C to about 130 °C, from about 75 °C to about 120 °C, from about 75 °C to about 110 °C, from about 75 °C to about 90 °C, or from about 75 °C to about 80 °C. In some embodiments, the heat apparatus is kept at a temperature within a range whose upper limit is about 35 °C, 40 °C, 45 °C, 50 °C, 60 °C, 70 °C, 75 °C, 80 °C, 90 °C, 100 °C, and whose lower limit is about 60 °C, 70 °C, 75 °C, 80 °C, 90 °C, 100 °C, 110 °C, 120 °C, 130 °C, 140 °C, 150 °C.

The heating apparatus is designed to heat only a portion of the cannula. For example, in certain embodiments, the heating apparatus only heats the tip of the cannula. In certain embodiments, the heating apparatus heats the last 1 cm, last 2 cm, last 3 cm, last 4 cm, or last 5 cm of the cannula. Thermal insulators may be used to prevent the heating of the remainder of the cannula. In certain embodiments, the heated tip of the cannula may be constructed of a different material than the remainder of the cannula so that the entire cannula is not heat by the heating apparatus. In certain embodiments, the entire length or substantially the entire length of the cannula is heated.

In certain embodiments, heating of the material progresses in two or more stages to make the material initially flowable at a first elevated temperature and then heated to a second elevated temperature to allow delivery to implantation sites. Temperature at a certain stage can be within the ranges disclosed above. In some embodiments, temperature at any stage can be 30 °C, 40 °C, 45 °C, 50 °C, 60 °C, 70 °C , 75 °C, 80 °C, 90 °C, 100 °C, 110 °C, 120 °C, 130 °C, 140 °C, 150 °C or above. In some embodiments, a first temperature is lower or higher than a second temperature. In some embodiments, temperature can be gradually increased along cannulas towards tips. In some embodiments, temperature at any stage may change at different time points. In some embodiments, temperature in cannulas at a certain stage can be at room temperature or below.

The cannula may be of any size. In certain embodiments, the length of the cannula ranges from approximately 1 cm to approximately 30 cm. In certain embodiments, the length of the cannula ranges from approximately 5 cm to approximately 20 cm. In certain embodiments, the length of the cannula ranges from approximately 1 cm to approximately 10 cm. In certain embodiments, the length of the cannula ranges from approximately 10 cm to approximately 20 cm. In certain embodiments, the length of the cannula ranges from approximately 20 cm to approximately 30 cm. The lumen of the cannula ranges in diameter from approximately 0.1 cm to approximately 2 cm. In certain embodiments, the diameter of the lumen ranges from approximately 0.25 cm to approximately 1 cm. In certain embodiments, the diameter of the lumen ranges from approximately 0.5 cm to approximately 1 cm. In certain embodiments, the diameter of the lumen ranges from approximately 1 cm to approximately 1.5 cm. In certain embodiments, the diameter of the lumen ranges from approximately 1.5 cm to approximately 2 cm. The size of the cannula may vary depending on the material to be administered through it. More viscous material or material with larger solid particles may be more easily delivered using a shorter cannula with a larger diameter. Material that is less viscous with no solid component such as a thermoplastic polymer may be delivered through a longer cannula with a narrower lumen. As would be appreciated by one of skill in the art, the dimensions of the cannula may be chosen by the medical professional using it based on a variety of factors including the characteristics of the material being administered, the size of the bony defect, the volume of material to be delivered, the surgical site, *etc.*

The tip of the cannula may be different than the remainder of the cannula. For example, the tip of the cannula may be widened, narrowed, flattened, *etc*. The cross-section of the tip may also be different than the cross-section of the remainder of the cannula. For example, the cross-section may be round, oval, square, rectangular, triangular, *etc*. Larger tips may be used to fill larger voids. Smaller tips may be used to deliver the material into smaller voids, or they may be used to more precisely deliver the material. The tip of the cannula is removable so that the tip is interchangeable.

The cannula may be flexible or rigid. In certain embodiments, the cannula is flexible. In other embodiments, the cannula is rigid. In certain embodiments, the tip of the cannula is rigid, and the remainder of the cannula is flexible. The cannula may be prepared from any suitable material. For example, the cannula may be made from metal, alloys, ceramics, plastics, polymers, glass, *etc.* In certain embodiments, the cannula is made of a material that can withstand sterilization (e.g., terminal radiation, electron beam irradiation, chemical sterilization, autoclaving etc.).

The cannula is optionally coated to prevent sticking of the material to the inside of the cannula. In certain embodiments, the interior of the cannula is coated with hydrophobic material. In certain embodiments, the coating is a hydrophobic polymeric coating. In certain embodiments, the coating comprises polytetrafluorethylene (TEFLON^{®}). In certain embodiments, the coating is a silicon-based material. In certain embodiments, the coating exudes a lubricant that aids in delivery of the material to be administered.

Material to be delivered such as a bone/polymer composite may be advanced through the inventive cannula using any advancing force. In certain embodiments, the advancing force is a direct mechanical force such as a plunger or piston which applies a force directly to the material. For example, in certain embodiments, a syringe loaded with the material to be delivered and connected to the cannula is used to advance the material through the cannula. In certain embodiments, a screw or auger is used to advance the material through the delivery device. In other embodiments, the advancing force is supplied via a pressurized gas or other fluid. The rate of flow through the cannula may be regulated by the amount of pressure applied to the material. By not heating the entire cannula, the amount of driving force necessary to advance the material is reduced. In certain embodiments, the rate of flow through the cannula may be regulated by varying the heating at the tip of the cannula. That is, the amount of heating at the tip of the cannula can be varied in order to control the flow rate of the material through the cannula for a given amount of advancing force or pressure.

In other embodiments, the present invention provides a device for delivering a heated material that is similar to a hot melt glue gun. The delivery device may have all or a portion of the following features: a cannula or barrel, heating element, power supply, battery, power cord, a trigger or plunger, a handle, a reservoir for the material, user controls, and casing.

The cannula and heating elements are as described herein. The handle, reservoir, controls, and casing provide for easy handling and use of the device based on the cannula design described above. The device provides for easy loading and administration of heated bone substitute material. The entire barrel or only the distal end of the barrel is heated by the heating element. Bone substitute material is advanced through the barrel based on pressure applied via the trigger. As the material advances into the heated portion of the barrel, it is heated and becomes flowable as it flows out of the device into the implant site. The device may be designed to operate at a specific temperature as determined by the material it will be used with, or the device may have a variable control so that the temperature can be adjusted by the user.

The inner components of the device are encased in an insulative material to prevent burning of the user. Any plastic, ceramic, silicone, or other insulative material may be used. Exemplary materials include a polyurethane, a polyester, a polyethylene, a polystyrene, a polyamide, nylone, polypropylene, polycarbonate, polyvinylchloride, polyvinylidenefluoride, polysulphone, polyretherimide, polyphenylenesulphone,polyphenylene sulphide, polyamide-imide, polyetheretherketone, polytetrafluoroethylene, polybenzimidazole, FOAMEX (polyurethane), KAPTON imide film, MYLAR (polyethylene terephthalate polyester film), a silicone-based material, or other thermally insulative material. In certain embodiments, cases of delivery devices are made of a heat-resistant plastic. The case allows for the device to be cool enough on the outside to be handled and to avoid burning the patient. The heating device is designed to maintain the barrel of the device at a temperature ranging from about 50 °C to about 120 °C. The case may include heat-insulating fins or honeycombing to insulating the heating elements or barrel. The case may also include warnings, serial number, instructions, lot number, logos, *etc*.

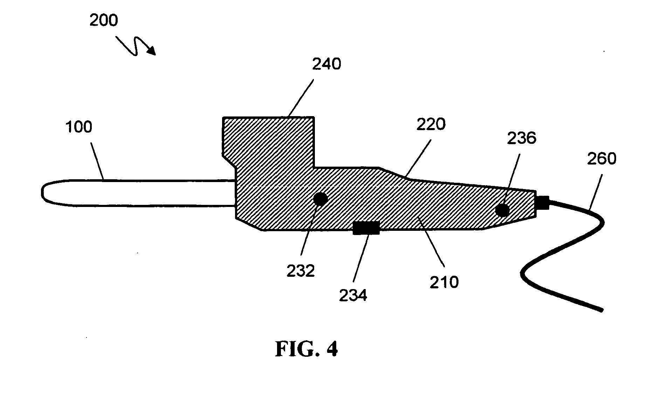

In one embodiment as shown in *Figures 3* and *4*, the tip of the cannula 100 is heated by pressing a heat control 232, *e.g.* a button or switch. The delivery device 200 may have an indicator light or digital display located on its body 210 to notify the user when the temperature of the tip of the cannula is within a desired operating range. The operating temperature of the device may be adjusted by a temperature control 236 and the desired operating temperature may be maintained by internal electronic circuitry utilizing information fed back from a temperature sensor 140 on the cannula 100. Once the temperature is within a desired range, the material feed control 234 is pressed to advance the material delivered from a chamber 240 into the cannula. In certain embodiments, material in the form of small beads or pellets is loaded into the chamber 240 prior to or during use of the device 200. In certain embodiments, a screw auger is disposed at the base of the chamber 240, and the screw auger extends to the near end of the cannula, or in some embodiments, into the cannula. The screw auger can be turned by an electric motor located within the body 210 of the device 200. In other embodiments, a plunger is disposed at the base of the chamber 240, and the plunger may be extended to the near end of the cannula. The screw auger or plunger may be activated by pressing the material feed control 234. As the screw auger turns or the plunger is advanced, the material within the chamber is fed into the cannula 100. As the cannula 100 fills with material, the auger or plunger applies pressure to the material so that the heated, flowable material at the end of the cannula is forced out of the cannula 100 and into the site of implantation.

### Methods

The present invention may be used in methods of heating bone substitute materials to a temperature wherein the material becomes flowable using an inventive device. The method includes providing the bone substitute material to be heated; placing the material in an inventive heating device; heating the material as it is advanced through a heated cannula or barrel of a delivery device; and administering the heated, flowable material to an implantation site. The steps of the method may be performed in a sterile environment such as an operating room.

Any bone substitute material may be heated to a flowable temperature using the methods. The material may become moldable at a temperature ranging from about 50 °C to about 120 °C are heated using the inventive methods. The material may become moldable at a temperature ranging from about 50 °C to about 100 °C. The material may become moldable at a temperature ranging from about 70 °C to about 95 °C. The material may become moldable at a temperature ranging from about 60 °C to about 90 °C. The material may become moldable at a temperature ranging from about 50 °C to about 75 °C. The material may become moldable at a temperature ranging from about 75 °C to about 100 °C. The material may become moldable at a temperature ranging from about 75 °C to about 95 °C. The material may become moldable at a temperature ranging from about 65 °C to about 75 °C. The material may become moldable at a temperature ranging from about 75 °C to about 85 °C. The material may become moldable at a temperature ranging from about 85 °C to about 95 °C. The bone substitute material may comprise bone particles and a biodegradable polymer. The material may comprise bone particles and polycaprolactone. The material may comprise bone particles and poly(lactide-co-glycolide). The material may comprise bone particles and poly(lactide). The material may comprise bone particle and poly(glycolide). The heating of the material using an inventive device may not substantially affect its ability to induce bone growth. That is, the cells, factors, proteins, or other bioactive agents in the material are not adversely affected by the heating of the material. The material may further comprise a porogen. The porogen may be a gas, liquid, or solid. The material may foam upon heating to form pores in the material. The porogen may be a substance that leaches from the material once it has been implanted. The material may comprise a pharmaceutical agent. The material may further comprise a growth factor or osteogenic factor.

The bone substitute material is typically packaged in packaging material for ease of handling. The packaging material may be any suitable packaging material for biological materials. Packaging materials may include plastic wraps, foils, paper, coated paper, coated foils, polymeric films, single metal foil, double metal foil, etc. Exemplary packaging materials include, but are not limited, to TYVEK, aluminum foil, and paper. The bone substitute material may be sterilized and then packaged. The packaging may be vacuum sealed or sealed under an inert atmosphere (*e.g.,* argon, nitrogen). The material may be packaged in several layers of packaging. The material may be supplied in various shapes and sizes for use in different heating devices. For example, the material may be supplied in cylinder form to be loaded into the barrel of a syringe or delivery device.

The bone substitute material is placed in an inventive heating device to heat it to the specified temperature as it is being delivered into the implantation site. An inventive heating device comprises a heated cannula as described herein. In certain embodiments, an inventive heating device is a hot melt delivery device.

The material is advanced through the heated device until it is sufficiently flowable for use in the orthopedic or dental application. Each bone substitute material has a characteristic transition temperature at which it will become flowable. The bone substitute material is heated to at least this characteristic temperature in the heating device. The material may not be much beyond this temperature so as not to affect the biological and/or chemical properties of the material. The material may not be heated beyond about 2-5 degrees Celsius above this transition temperature. The material is typically heated as it is about to exit the heating device. Therefore, the material is not kept heated for an substantial length of time. The material may be heated for less than 1 minute. The material may be heated for less than 30 seconds.

As the flowable, heated material exits the device, it is delivered to the implantation device. The heated material is typically implanted by a surgeon or other health care professional. As the material cools after implantation, it becomes set and able to bear a physiological load. The heated tip of the inventive device may also be used to remelt and manipulate material that has previously been implanted and set. This may be done during the same implantation procedures or during a revision procedure at a later date.

By adding a vacuum or suction to the non-heated end of the cannula, it may be used to remove or aspirate portions of material through the cannula. The cannula may also be used to melt a portion of an implant, pull the melted portion into the cannula, allow it resolidify, and then use the cannula as a handle to remove or reposition the attached implanted material. Different sizes or shapes of tips may be used to remelt and reposition implanted material. In certain embodiments, the tip used to reposition implanted material may have no orifice.

The material may be used for any orthopedic or dental application. The subject is typically a patient with a disorder or disease related to the skeletal system including bones and joints. The subject may have a bony defect such as a fracture. The subject is typically a mammal although any animal with bones may benefit from treatment with the inventive composite. The subject may be a vertebrate (*e.g*., mammals, reptiles, fish, birds, *etc.*). The subject may be a human. The subject may be a domesticated animal such as a dog, cat, horse, etc. Any bone or joint disease or disorder may be treated using the inventive composite including genetic diseases, congenital abnormalities, fractures, iatrogenic defects, bone cancer, bone metastases, inflammatory diseases (*e.g.* rheumatoid arthritis), autoimmune diseases, metabolic diseases, and degenerative bone disease (*e.g*., osteoarthritis). The bone substitute material may be formulated for the repair of a simple fracture, compound fracture, or non-union; as an external fixation device or internal fixation device; for joint reconstruction, arthrodesis, arthroplasty, or cup arthroplasty of the hip; for femoral or humeral head replacement; for femoral head surface replacement or total joint replacement; for repair of the vertebral column, spinal fusion or internal vertebral fixation; for tumor surgery; for deficit filling; for discectomy; for laminectomy; for excision of spinal tumors; for an anterior cervical or thoracic operation; for the repairs of a spinal injury; for scoliosis, for lordosis or kyphosis treatment; for intermaxillary fixation of a fracture; for mentoplasty; for temporomandibular joint replacement; for alveolar ridge augmentation and reconstruction; as an inlay osteoimplant; for implant placement and revision; for sinus lift; for a cosmetic procedure; for revision surgery; for revision surgery of a total joint arthroplasty; and for the repair or replacement of the ethmoid, frontal, nasal, occipital, parietal, temporal, mandible, maxilla, zygomatic, cervical vertebra, thoracic vertebra, lumbar vertebra, sacrum, rib, sternum, clavicle, scapula, humerus, radius, ulna, carpal bones, metacarpal bones, phalanges, ilium, ischium, pubis, femur, tibia, fibula, patella, calcaneus, tarsal bones, or metatarsal bones. The heated bone substitute material may be used to seal a defect, void, or hole in a bone. For example, a bony defect may be filled with mineralized and/or partially or fully demineralized allograft bone or other bone substitute material, and the defect is sealed with the heated bone substitute material using an inventive device and/or method.

After implantation, the bone substitute material typically stays at the site of implantation and is gradually resorbed by the body as bone forms in and around it. The material is typically engineered to provide the mechanical strength necessary for the implantation site. The material may be resorbed after approximately 1 month to approximately 6 years. The resorption rate will depend on the polymer used in the composite, the site of implantation, the patient, disease condition, etc. The osteoimplant may last from approximately 1 month to approximately 6 months. The osteoimplant may last from approximately 6 months to approximately 1 year. The osteoimplant may last from approximately 1-2 years. The osteoimplant may last from approximately 2-3 years. The osteoimplant may last from approximately 5 years.

### Equivalents and Scope

The foregoing has been a description of certain non-limiting preferred embodiments of the invention. Those skilled in the art will recognize, or be able to ascertain using no more than routine experimentation, many equivalents to the specific embodiments of the invention described herein. Those of ordinary skill in the art will appreciate that various changes and modifications to this description may be made without departing from the scope of the present invention, as defined in the following claims.

## Claims

1. A cannula device (100) for delivering a heated bone substitute material, wherein a tip (110) of the cannul comprises resistive material and is heated sufficiently to make the material flowable as it passes through the tip, wherein the cannula device (100) comprises
(i) a conductive element (122) for providing electrical current to the tip (110) and
(ii) a conductive element (124) for receiving electrical current from the tip (110), **characterised in that** the tip (110) is removable and interchangeable.

2. A device (200) for delivering a heate bone substitute material comprising:
a barrel (240) for receiving and holding material to be dispensed;
the cannula device (100) of claim 1 through which the material is dispensed, wherein the cannula is in communication with the barrel (240) ; and
a dispenser (234) for dispensing the material through the cannula.

3. The device (200) of claim 2 further comprising:
a syringe (240) for holding the unheated material; or
a means (234) for regulating the flow of material through the cannula;
a reservoir (240) for holding the unheated material; and
optionally, a grip (220) for holding the device. device claim

4. The device (200) of claim 2, further comprising:
a gun body (210);
A reservoir (240) for holding the unheated material, wherein the reservoir (240) is in communication with a lumen of the cannula; and
optionally, a grip (220).

5. The device (100; 200) of claim 1, 2, 3 or 4, wherein the cannula is heated in two or more stages to make the material flowable at a first temperature and heated to a second temperature.

6. The device (200) of claim 3 or 4, wherein the device comprises a grip (220) and the grip (220) comprises a trigger mechanism; or wherein the device (200) further comprises a power cord (260).

7. The device (100; 200) of claim 1, 2, 3 or 4, wherein only the tip (110) of the cannula is heated.

8. The device (100; 200) of claim 1, 2, 3 or 4, wherein the tip (110) of the cannula is flattened.

9. The device (100; 200) of claim 1, 2, 3 or 4, wherein the tip (110) of the cannula is widened; or wherein the tip (110) of the cannula is narrowed.

10. The device (100; 200) of claim 1, 2, 3 or 4, wherein the device comprises two or more heated tips (110).

11. The device (100; 200) of claim 1, 2, 3 or 4, wherein the cannula comprises a thermal insulator between the heated tip (110) and a unheated portion of the cannula.

12. The device (100; 200) of claim 1, 2, 3 or 4, wherein the material is a bone particle/polymer composite.

13. The device (100; 200) of claim 1, 2, 3 or 4, wherein the material is a thermoplastic composite or a thermoplastic polymer.

14. The device (100; 200) of claim 1, 2, 3 or 4, wherein the tip (110) of the cannula is heated to a temperature ranging from 45 °C to 130 °C; wherein, optionally, the tip (110) of the cannula is heated to a temperature ranging from 75 °C to 110 °C.

15. A kit comprising:
a device (200) or cannula (100) of claim 1, 2, 3 or 4; and
a bone substitute material.

## Patentansprüche

1. Kanülenvorrichtung (100) zum Zuführen eines erhitzten Knochenersatzmaterials, wobei eine Spitze (110) der Kanüle ein resistives Heizmaterial aufweist und ausreichend erhitzt wird, um das Material fließfähig zu gestalten, wenn es die Spitze durchquert, wobei die Kanülenvorrichtung (100) Folgendes umfasst:
(i) ein leitfähiges Element (122) zum Liefern von elektrischem Strom an die Spitze (110) und
(ii) ein leitfähiges Element (124) zum Aufnehmen von elektrischem Strom von der Spitze (110),
**dadurch gekennzeichnet, dass** die Spitze (110) entfernt und ausgetauscht werden kann.

2. Vorrichtung (200) zum Zuführen eines erhitzten Knochenersatzmaterials, die Folgendes umfasst:
einen Zylinder (240) zum Aufnehmen und Halten von auszugebendem Material;
die Kanülenvorrichtung (100) nach Anspruch 1, durch die das Material ausgegeben wird, wobei die Kanüle mit dem Zylinder (240) in Verbindung steht; und einen Spender (234) zum Ausgeben des Materials durch die Kanüle.

3. Vorrichtung (200) nach Anspruch 2, die weiter Folgendes aufweist: entweder eine Spritze (240) zum Halten des nicht erhitzten Materials; oder
ein Mittel (234) zum Regeln des Materialflusses durch die Kanüle;
einen Behälter (240) zum Halten des nicht erhitzten Materials; und
optional, einen Griff (220) zum Halten der Vorrichtung.

4. Vorrichtung (200) nach Anspruch 2, die weiter Folgendes aufweist:
einen Pistolenkörper (210);
einen Behälter (240) zum Halten des nicht erhitzten Materials, wobei der Behälter (240) mit einem Lumen der Kanüle in Verbindung steht; und
optional, einen Griff (220).

5. Vorrichtung (100, 200) nach Anspruch 1, 2, 3 oder 4, bei der die Kanüle in zwei oder mehr Stadien erhitzt wird, um das Material bei einer ersten Temperatur fließfähig und auf eine zweite Temperatur erhitzt zu gestalten.

6. Vorrichtung (200) nach Anspruch 3 oder 4, wobei die Vorrichtung einen Griff (220) aufweist, und der Griff (220) einen Auslösemechanismus aufweist, oder wobei die Vorrichtung (200) weiter ein Stromkabel (260) aufweist.

7. Vorrichtung (100; 200) nach Anspruch 1, 2, 3 oder 4, bei der nur die Spitze (110) der Kanüle erhitzt wird.

8. Vorrichtung (100; 200) nach Anspruch 1, 2, 3 oder 4, bei der die Spitze (110) der Kanüle abgeflacht ist.

9. Vorrichtung (100; 200) nach Anspruch 1, 2, 3 3 oder 4, bei der die Spitze (110) der Kanüle erweitert ist; oder bei der die Spitze (110) der Kanüle verengt ist.

10. Vorrichtung (100; 200) nach Anspruch 1, 2, 3 oder 4, wobei die Vorrichtung zwei oder mehr erhitzte Spitzen (110) aufweist.

11. Vorrichtung (100; 200) nach Anspruch 1, 2, 3 oder 4, bei der die Kanüle einen Wärmeisolator zwischen der erhitzten Spitze (110) und einem nicht erhitzten Teil der Kanüle aufweist.

12. Vorrichtung (100; 200) nach Anspruch 1, 2, 3 oder 4, bei der das Material einen Verbundstoff aus Knochenpartikeln und Polymer darstellt.

13. Vorrichtung (100; 200) nach Anspruch 1, 2, 3 oder 4, bei der das Material einen thermoplastischen Verbundstoff oder ein thermoplastisches Polymer darstellt.

14. Vorrichtung (100; 200) nach Anspruch 1, 2, 3 oder 4, bei der die Spitze (110) der Kanüle auf eine Temperatur im Bereich von 45°C bis 130°C erhitzt wird, wobei die Spitze (110) der Kanüle optional auf eine Temperatur im Bereich von 75°C bis 110°C erhitzt wird.

15. Instrumentensatz, der Folgendes aufweist:
eine Vorrichtung (200) oder Kanüle (100) nach Anspruch 1, 2, 3 oder 4; und
ein Knochenersatzmaterial.

## Revendications

1. Dispositif de type canule (100) à des fins d'administration d'un matériau de substitut osseux chauffé, dans lequel une extrémité (110) de la canule comporte un matériau chauffant résistif et est chauffée suffisamment pour rendre le matériau fluide alors qu'il passe au travers de l'extrémité, dans lequel le dispositif de type canule (100) comporte
(i) un élément conducteur (122) destiné à fournir un courant électrique au niveau de l'extrémité (110) et
(ii) un élément conducteur (124) destiné à recevoir un courant électrique en provenance de l'extrémité (110),
**caractérisé en ce que** l'extrémité (110) est amovible et interchangeable.

2. Dispositif (200) à des fins d'administration d'un matériau de substitut osseux chauffé comportant :
un cylindre (240) destiné à recevoir et à contenir le matériau devant être distribué ;
le dispositif de type canule (100) selon la revendication 1 au travers duquel le matériau est distribué, dans lequel la canule est en communication avec le cylindre (240) ; et
un distributeur (234) destiné à distribuer le matériau au travers de la canule.

3. Dispositif (200) selon la revendication 2, comportant par ailleurs :
soit
une seringue (240) destinée à contenir le matériau non chauffé ; soit un moyen (234) destiné à réguler le débit du matériau au travers de la canule ;
un réservoir (240) destiné à contenir le matériau non chauffé ; et éventuellement, un manche (220) pour tenir le dispositif.

4. Dispositif (200) selon la revendication 2, comportant par ailleurs :
un corps de pistolet (210) ;
un réservoir (240) destiné à contenir le matériau non chauffé, dans lequel le réservoir (240) est en communication avec une lumière de la canule ; et
éventuellement, un manche (220).

5. Dispositif (100 ; 200) selon l'une quelconque des revendications 1, 2, 3 ou 4, dans lequel la canule est chauffée en deux ou plusieurs étapes pour rendre le matériau fluide à une première température et chauffée à une seconde température.

6. Dispositif (200) selon la revendication 3 ou la revendication 4, dans lequel le dispositif comporte un manche (220) et le manche (220) comporte un mécanisme de déclenchement ; ou dans lequel le dispositif (200) comporte par ailleurs un cordon d'alimentation (260).

7. Dispositif (100 ; 200) selon l'une quelconque des revendications 1, 2, 3 ou 4, dans lequel seule l'extrémité (110) de la canule est chauffée.

8. Dispositif (100 ; 200) selon l'une quelconque des revendications 1, 2, 3 ou 4, dans lequel l'extrémité (110) de la canule est aplatie.

9. Dispositif (100 ; 200) selon l'une quelconque des revendications 1, 2, 3 ou 4, dans lequel l'extrémité (110) de la canule est élargie ; ou dans lequel l'extrémité (110) de la canule est rétrécie.

10. Dispositif (100 ; 200) selon l'une quelconque des revendications 1, 2, 3 ou 4, dans lequel le dispositif comporte deux ou plusieurs extrémités chauffées (110).

11. Dispositif (100 ; 200) selon l'une quelconque des revendications 1, 2, 3 ou 4, dans lequel la canule comporte un isolant thermique entre l'extrémité chauffée (110) et une partie non chauffée de la canule.

12. Dispositif (100 ; 200) selon l'une quelconque des revendications 1, 2, 3 ou 4, dans lequel le matériau est un composite de particules d'os/de polymère.

13. Dispositif (100 ; 200) selon l'une quelconque des revendications 1, 2, 3 ou 4, dans lequel le matériau est un composite thermoplastique ou un polymère thermoplastique.

14. Dispositif (100 ; 200) selon l'une quelconque des revendications 1, 2, 3 ou 4, dans lequel l'extrémité (110) de la canule est chauffée à une température allant de 45°C à 130°C ; dans lequel, éventuellement, l'extrémité (110) de la canule est chauffée à une température allant de 75°C à 110°C.

15. Kit comportant :
un dispositif (200) ou canule (100) selon l'une quelconque des revendications 1, 2, 3 ou 4 ; et
un matériau de substitut osseux.
